# EUROPEAN PATENT SPECIFICATION

(11) **EP 2 429 537 B1**
(45) Date of publication and mention of the grant of the patent: **16.07.2014**
(21) Application number: 10718269.3
(22) Date of filing: 11.05.2010
(51) Int. Cl.: A61K 35/56, A61P 25/22

(54) **A FERTILIZED, INCUBATED AVIAN EGG OR EXTRACT FOR THE TREATMENT OF STRESS**
BEFRUCHTETES UND INKUBIERTES VOGELEI ODER EXTRAKT DAVON ZUR BEHANDLUNG VON STRESS
OEUF AVAIRE FERTILIZE ET INCUBE OU EXTRAIT POUR LE TRAITEMENT DU STRESS

(30) Priority: 11.05.2009 GB 0908101
(43) Date of publication of application: 21.03.2012
(73) Proprietor: Med-EQ AS, 3111 Tønsberg (NO)
(72) Inventor: MORTENSEN, Nils Christian, N-3111 Tonsberg (NO); BAKKE, Kjell Hans, N-3111 Tonsberg (NO)
(74) Representative: Campbell, Neil Boyd
(86) International application number: PCT/GB2010/000930
(87) International publication number: WO 2010/130980

(56) References cited:
- US-A- 5 641 517
- US-A1- 2001 033 869
- SCHULT J ET AL: "Effects of powdered fertilized eggs on the stress response" CLINICAL NUTRITION, CHURCHILL LIVINGSTONE, LONDON, GB, vol. 29, no. 2, 1 April 2010 (2010-04-01), pages 255-260, XP026985313 ISSN: 0261-5614 [retrieved on 2009-10-17]
- Swanson Vitamins: "Dr. Eskeland's youth tissue exctract- Norway's "Advanced Vitality" formula."[Online] 1 August 2006 (2006-08-01), XP002605452 Retrieved from the Internet: URL:http://www.swansonvitamins.com/health- library/products/youth-tissue-extract.html > [retrieved on 2010-10-11]
- CHAUDIEU ET AL: "Abnormal reactions to environmental stress in elderly persons with anxiety disorders: Evidence from a population study of diurnal cortisol changes" JOURNAL OF AFFECTIVE DISORDERS, ELSEVIER BIOCHEMICAL PRESS, AMSTERDAM, NL LNKD- DOI:10.1016/J.JAD.2007.07.025, vol. 106, no. 3, 16 January 2008 (2008-01-16), pages 307-313, XP022423162 ISSN: 0165-0327
- ARMARIO A ET AL: "Acute stress markers in humans: Response of plasma glucose, cortisol and prolactin to two examinations differing in the anxiety they provoke" PSYCHONEUROENDOCRINOLOGY, OXFORD, GB LNKD- DOI:10.1016/0306-4530(95)00048-8, vol. 21, no. 1, 1 January 1996 (1996-01-01), pages 17-24, XP009103666 ISSN: 0306-4530
- All Natural Pharma: "Young Tissue Extract"[Online] 1996, XP002605453 Retrieved from the Internet: URL:http://www.memomax.no/YTE.html> [retrieved on 2010-10-11]
- DATABASE WPI Week 200667 Thomson Scientific, London, GB; AN 2006-641928 XP002605454 -& JP 2006 249045 A (KANEBO LTD) 21 September 2006 (2006-09-21)

## Description

This invention relates to the use of fertilised eggs, e.g. hen's eggs, or extracts thereof in the treatment of stress, such as perceived stress as well as in the use of such eggs to normalise cortisol response in chronically stressed individuals. The invention further relates to the use of the eggs or an extract thereof in the treatment of anxiety in chronically stressed individuals.

### Background of Invention

The avian egg contains a multitude of the proteins, lipids, vitamins, minerals, and growth factors. There are also additional defense factors contained to protect against bacterial and viral infection and biologically active components, making it more than just a source of nutrients. Intake of egg components has been associated with biological activities like novel antimicrobial activities, immunomodulatory responses, anticancer activity, and antihypertensive activities, antioxidant properties, protease inhibition, and nutrient bioavailability.

Historically, when chickens were kept in natural conditions and cockerels and hens were kept together many eggs consumed were fertilized. In these eggs, and before the advent of the refrigerator, a process took place in the eggs whereby the amino acid chains (peptides) were changed before becoming an embryo. There are therefore significant differences in terms of chemistry between an unfertilised and fertilised hen's egg.

Whilst the components that are found do not apparently differ significantly from ordinary eggs, a fertilised egg grows an embryo and must therefore be quite different. Fertilised eggs can contain amino acids such as tyrosine, phenylalanine, methionine, aspartic acid, cystine, treonine, serine, glutamic acid, proline, glycine, alanine, valine, isoleucine, leucine, histidine, ornitine, lysine, arginine, hyroxyproline and ammonia, in addition to vital vitamins as vitamin A (retinol), vitamin B8 (biotin) and vitamin B9 (folate). The amounts of the different amino acids vary somewhat between the samples.

The use of a fertilised egg to treat various conditions is known. In US 5641517, the use of a fertilised egg extract is suggested for increasing sexual potency and to elevate testosterone levels. In US 2001/033869, a fertilised egg extract is suggested for use in the treatment of depression.

The present inventors have realised that a fertilised egg, in particular an extract from a fertilised egg, can have further physiological benefits, in particular in reducing stress, such as the perception of stress, and reducing anxiety in all individuals (especially in chronically stressed individuals) and normalising cortisol levels in chronically stressed and non stressed individuals in response to acute stress.

Many people now suffer from chronic stress. Chronic stress is a state of ongoing physiological arousal. This occurs when the body experiences so many stressors that the autonomic nervous system rarely has a chance to activate the relaxation response. This type of chronic stress response occurs all too frequently in our modern lifestyle, when everything from high-pressured jobs to loneliness can keep the body in a state of perceived threat and chronic stress. In this case, our "fight-or-flight" response, which was designed to help us fight a few life-threatening situations spaced out over a long period can wear down our bodies and cause us to become ill.

Treatment for chronic stress, especially in the most severe cases, often involves removal of the individual from the stressful situation, e.g. giving up a stressful job and allowing some time for recuperation. However, this option is not always available to an individual.

One of the problems with chronically stressed individuals is that their ability to deal with situations which they perceive as stressful is also compromised. People with chronic stress often have a suppressed cortisol response to acute stress which in turn limits their ability to react appropriately.

The inventors have surprisingly found that a fertilised egg or an extract from a fertilised egg is able to normalise the response of a chronically stressed individual to an acutely stressful situation so that their endocrine response is more natural and appropriate to the acute stimulus. The use of the extract can actually therefore increase their cortisol response.

Cortisol is a corticosteroid hormone or glucocorticoid produced by the adrenal cortex, which is part of the adrenal gland (in the Zona fasciculata and the Zona reticularis of the adrenal cortex). It is usually referred to as the "stress hormone" as it is involved in response to stress and anxiety. It increases blood pressure and blood sugar, and reduces immune responses.

In surprising contrast, in individuals who are not chronically stressed, from hereon normal individuals, the use of the fertilised egg can cause a reduction in cortisol response during acute stress. Whilst too little cortisol leads to an inappropriate reaction to acute stress, too much cortisol can cause panic in an individual subject to acute stress when calmness and sensible thinking is required.

Overall therefore, the extract has the remarkable effect of normalizing cortisol response, i.e. increasing the levels of cortisol in chronically stressed individuals but reducing levels in normal individuals (relative in both cases to individuals not taking the fertilised egg), in response to an acutely stressful event.

Acute stress describes stress reactions which occur occasionally but are short lived. Again, these acutely stressful events can vary from a traumatic event to sitting an important examination.

Whilst there are physiological ways of measuring responses to stress, an important aspect of any stress management regime is the perceived stress of an individual. Some individuals cope better with stress than others and it is inevitable therefore that cortisol levels are not the only indicator of stress. Perhaps more significant is the perception of the individual.

The inventors have also found that the fertilised egg extract when given to individuals results in a reduction of perceived stress in that individual. High cortisol release during acute stress does not correlate with high perceived stress and studies show that these reactions are independent of each others.

The measurement of physiological stress reaction and perceived psychological stress is achieved using a Trier Social Stress Test (TSST). Although stress has been described as a non-specific response of the body, it is possible to discern specific endocrine stress responses caused by specific emotional reactions to novel, ambivalent or uncontrollable situations and stimuli. Social stress induces elevated cortisol levels, particularly if the stressor is perceived as uncontrollable, unpredictable, and constitutes a social-evaluative threat due to the judgment of others. The hypothalamic-pituitary-adrenal (HPA) axis plays a major role in the response to this kind of stressors with a robust increase of ACTH and cortisol.

An analysis of 208 laboratory studies of acute psychological stressors showed that the TSST is the best standardized and most efficient psychological stress protocol in humans.

With respect to psychological parameters, the TSST leads to a moderate rise in fear. The biological response comprehends an increase of adrenocorticotropin hormone (ACTH), cortisol, prolactin, growth hormone, norepinephrine, epinephrine, heart rate and blood pressure.

A further benefit of the invention is in anxiety treatment. Anxiety is a psychological and physiological state characterized by cognitive, somatic, emotional, and behavioural components. These components combine to create an unpleasant feeling that is typically associated with uneasiness, fear, or worry.

Anxiety is a generalized mood state that occurs without an identifiable triggering stimulus. As such, it is distinguished from fear, which occurs in the presence of an external threat. Additionally, fear is related to the specific behaviours of escape and avoidance, whereas anxiety is the result of threats that are perceived to be uncontrollable or unavoidable.

Anxiety is a normal reaction to stress. It may help a person to deal with a difficult situation, for example at work or at school, by prompting one to cope with it.

Anxiety is a separate indication from depression. Individuals with clinical depression do not necessary develop anxiety and vice versa.

### Summary of Invention

Thus viewed from one aspect the invention provides a composition comprising a fertilised, incubated avian egg or extract therefrom for use in the treatment of stress such as perceived stress in a chronically stressed individual.

Viewed from another aspect the invention provides a composition comprising a fertilised, incubated avian egg or extract therefrom for use in normalising the cortisol response of an individual to acute stress in a chronically stressed individual.

Viewed from another aspect the invention provides a composition comprising a fertilised, incubated avian egg or extract therefrom for use in the treatment of anxiety in a chronically stressed individual.

### Definitions

By a chronically stressed individual is meant an individual who has been diagnosed with chronic stress by a standardized questionnaire called TICS ("Trierer Inventory for Chronic Stress"; Schulz, Schlotz & Becker, 2004) assessing the subjective perception of stress load during the past three months. Subjects rate how often they experienced situations characterizing chronic stress. Summarized, the items describe 10 scales: work overload, social responsibility, overextended at work, social failure, aversive work load, social conflicts, performance pressure at work, performance pressure in social situations, social isolation, worry propensity. The questionnaire also yields a sum score, which consists of the first 9 subscales.

This questionnaire has been translated to several languages and is widely used for diagnosing chronic stress levels. Among others, the TICS is well approved in health research and work psychology. The test manual has a norm sample distribution and subjects > 50th percentile are regarded as chronically stressed.

Stress such as the perception of stress is measured using Visual Analogue Scales (VAS) in a well established laboratory stress test protocol for humans, the Trier Social Stress Test (TSST) as described herein (see also Kirschbaum, Pirke & Hellhammer, 1994). Since, the combination of VAS and TSST has become a well known test procedure for assessing stress and is used by many clinical studies all over the world. An analysis of Dickerson & Kemeny in 2004 compared 208 laboratory studies of psychological stressors. The analysis showed that the TSST is the best standardized and most efficient psychological stress protocol. The 15 min protocol includes a 5 min introduction and preparation phase followed by a 5 min job interview and 5 min mental arithmetic in front of an audience. To assess subjects' perception of this acute stressor they are asked to rate their level of stress three times: right before, in the middle and immediately after the TSST. On a bipolar dimension ("low" to "high") their perceived stress load is being marked.

By anxiety is meant a diagnosis with the State-Trait Anxiety Inventory (STAI; "State-Trait-Angstinventar", Laux, Glanzmann, Schaffner, & Spielberger, 1981) which is the German version of the STAI developed by Spielberger and colleagues in 1970. The two scales with 20 items assess anxiety as a state (STAI-X1). State anxiety describes an emotional state characterized by tension, worrying, nervousness, agitation, fear of future events and an increased activity of the autonomic nervous system (Laux, et al., 1981). For state anxiety they have to rate statements how they feel currently ("not at all", "a bit", "quite a lot", "very much so").

The STAI-X1 is a well validated and reliable tool for assessment of state anxiety and therefore part of the standard TSST-protocol for many years. Subjects fill in the questionnaire twice, once immediately before and once after the TSST.

Stress triggers a reaction of the Hypothalamus-Pituitary-Adrenal-Axis (HPAA) and releases a cascade of hormones: CRF > ACTH > Cortisol. This system is regulated by a feedback mechanism. Chronic stress in humans means receptors are being down regulated. In an acute stressfully situation down regulated receptors react with a reduced cortisol secretion whereas non-stressed subjects react with a normal and high cortisol response. By normalisation of cortisol response when subject to an acute stress is meant that in chronic stressed subjects cortisol levels are raised and in non stressed subjects cortisol levels are rather dampened.

By an acute stress is meant a stress caused by an acute event characterized by novelty, ego-involvement, anticipation, uncontrollability, and unpredictability. These key elements defined by Mason in 1968 reliably provoke a reaction of the endocrine and the autonomic nervous system as well as subject's perceived stress and well being. These key elements are integrated in the TSST and reliably provoke a rise in ACTH and cortisol, in heart rate and perceived stress, and a decrease in mood and wellbeing, respectively.

It will be appreciated that individuals being treated according to the invention are human and can be of any sex or age. Preferably the individual is an adult, especially one of more than 18 years of age.

Avian eggs used in the invention preferably derive from birds bred for egg production, e.g. hens, geese, ducks, quail, turkeys, ostriches, pheasants, pigeons or the like, most especially hens.

As shown by the trials reported below, the effect has been demonstrated with fertilized and incubated eggs rather than with unfertilized and/or unincubated eggs. It is believed that the results obtained are, in part, the result of the production of the active factors in the transformation of the egg yolk during embryogenesis.

In the general production of eggs for human consumption, the eggs used are unfertilized. Even if a fertilized egg is inadvertently presented for human consumption, these will generally be unincubated eggs or eggs which have been incubated for a maximum of 1 or 2 days.

The eggs used according to the invention are desirably ones in the blastodermal and subsequent preembryonic to protoembryonic stages in which yolk transformation has begun, but the organs of the embryo are barely if at all discernible; this corresponds essentially to the subembryonic liquid stage of embryogenesis (generally 3 to 14 days incubation for a hen's egg), or the period up to the acceleration of calcium uptake by the embryo (this occurs after about 15 days incubation for the hen's egg).

In the case of fertilized hens' eggs used according to the invention, the incubation period is preferably 2 to 15 days especially 3 to 12, particularly 5 to 10, and most preferably about 9 or about 10 days. Eggs incubated for such periods would generally not be considered fit for human consumption due to the degree of transformation of the yolk that has occurred and, for the upper incubation limit, due to the presence of an embryo with visible organs.

It is accepted that fertilized eggs may have been used in the past as a foodstuff since the nutritional value of eggs is well known. Such eggs would not have been incubated for a prolonged period and the efficacy of fertilized incubated eggs in the conditions discussed herein has not previously been recognized.

The composition of the invention can be formed from the whole egg (including shell although preferably this will be removed) or just from an extract of the egg. Preferably, an extract of an egg is employed, e.g., a dried extract. A dry egg extract is light, easy to transport and easy to formulate into a dosage form making it an ideal material for sale to consumers.

The material formed after freeze drying may need to be ground to form a powder of appropriate particle size.

The dried egg extract may be prepared for example by freeze drying the whole uncooked contents from within the egg shell.

In a preferred embodiment the contents of a fertilised and incubated egg may be divided to remove some of the components. It has been found that the active components required to effect the invention are primarily located within the solid parts of the egg and hence removal of other parts of the fertilised egg is possible.

If desired some or all of the liquid components within the egg can be removed. In a further embodiment the extract from the egg is made simply from the preembryo component of the egg.

After isolation of the desired part of the egg, this can be frozen for future use. Parts of the frozen extract can be removed intermittently for use, milled and freeze dried as discussed above to form a composition of the invention.

A highly preferred embodiment therefore involves the use of a fertilised, incubated, preembryo powder in the invention. In this embodiment, the composition of the invention utilises an extract from a hen's egg which comprises oligopeptides which have been separated from the total mass of the egg.

Freeze drying can be effected using conventional techniques and at conventional temperatures e.g. treated at 40 to 70°C, such as 50 to 60°C for 1 to 50 hours, e.g. 15 to 40 hrs.

The freeze dried egg product produced in this way is low in cholesterol and, as long as the eggs' surfaces are sterilized before removal of the contents there should be no health concerns relating to the ingestion of the product. In a further embodiment therefore, the egg is sterilised before removal of its contents.

An egg extract of the invention may contain protein, fat, carbohydrate, water, ash, tyrosine, phenylalanine, methionine, aspartic acid, cystine, threonine, serine, glutamic acid, proline, glycine, alanine, valine, isoleucine, leucine, histidine, ornitine, lysine, arginine, hyroxyproline, ammonia, vitamin A, folic acid and biotine.

Viewed from a still further aspect, the invention also provides a process for the preparation of a fertilised, incubated egg composition of use in this invention comprising incubating fertilized avian eggs into the blastodermal to protoembryonic stage (e.g. for 2-15, preferably about 10 days in the case of hens eggs) and freeze drying the shell contents or a component thereof, especially the preembryo component thereof.

The process for the formation of the composition of use in the invention in a straightforward embodiment comprises incubating fertilized hens eggs for about 10 days, cracking them open, freeze drying the contents, grinding the resultant product to a powder, and admixing the powder with any desired physiologically tolerable additives. '

More particularly the process involves removal of all liquid contents of the egg before freeze drying. An egg extract made in this fashion forms a still yet further aspect of the invention which therefore provides an egg extract obtained by incubating a fertilized avian egg into the blastodermal to protoembryonic stage (e.g. for 2-15, preferably about 10 days in the case of hens eggs), removing the shell and all liquid contents from the egg and freeze drying residue.

The composition of the invention can be used to treat anxiety, stress such as perceived stress and to normalise cortisol response in an acutely stressful environment.

By treating or treatment is meant at least one of:
(i). preventing or delaying the appearance of clinical symptoms of the condition developing;
(ii). inhibiting the condition, i.e. arresting, reducing or delaying the development of the condition or a relapse thereof or at least one clinical or subclinical symptom thereof, or
(iii). relieving or attenuating one or more of the clinical or subclinical symptoms of the condition.

The benefit to a subject to be treated is either statistically significant or at least perceptible to the individual or to the physician. In general, a skilled man can appreciate when "treatment" occurs.

The word "treatment" is also used herein to cover prophylactic treatment, i.e. treating subjects who are at risk of developing a condition in question.

In order to treat a disease an effective amount of the composition needs to be administered to an individual. A "therapeutically effective amount" means the amount of a compound that, when administered for treating a condition, is sufficient to effect such treatment. The "therapeutically effective amount" will vary depending on the composition, the condition and its severity and the age, weight, physical condition and responsiveness of the subject to be treated and will be ultimately at the discretion of a doctor.

For use according to the invention it is of course feasible to administer the egg composition without any extensive preparation, e.g. whisked into a glass of milk. However, a freeze dried egg composition has a shelf life which facilitates manufacture, packaging, transport and storage of the compositions according to the invention and by preference such compositions will be administered.

The compositions of the invention are preferably in pulverulent form, optionally including other components serving for example to enhance or mask flavour or to facilitate dispersion of the egg powder in an aqueous fluid for oral administration.

The egg powder preferably is present at 5 to 90% by weight, particularly 10 to 80% by weight, especially preferably about 25 to 75% by weight in any composition being administered.

While it is possible that, for use according to the invention, a composition of the invention may be administered as the bulk substance, it is preferable to present the active ingredient in a pharmaceutical formulation, for example, wherein the agent is in admixture with a pharmaceutically acceptable carrier selected with regard to the intended route of administration and standard pharmaceutical practice.

The term "carrier" refers to a diluent, excipient, and/or vehicle with which an active compound is administered. The pharmaceutical compositions of the invention may contain combinations of more than one carrier. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition. The choice of pharmaceutical carrier can be selected with regard to the intended route of administration and standard pharmaceutical practice. The pharmaceutical compositions may comprise as, in addition to, the carrier any suitable binder(s), lubricant(s), suspending agent(s), coating agent(s), and/or solubilizing agent(s).

A "pharmaceutically acceptable excipient" means an excipient that is useful in preparing a pharmaceutical composition that is generally safe, non-toxic and neither biologically nor otherwise undesirable, and includes an excipient that is acceptable for human pharmaceutical use. A "pharmaceutically acceptable excipient" as used in the present application includes both one and more than one such excipient.

It will be appreciated that pharmaceutical compositions for use in accordance with the present invention may be in the form of oral, parenteral, transdermal, inhalation, sublingual, topical, implant, nasal, or enterally administered (or other mucosally administered) suspensions, capsules or tablets, which may be formulated in conventional manner using one or more pharmaceutically acceptable carriers or excipients.

There may be different composition/formulation requirements depending on the different delivery systems. Likewise, if the composition comprises more than one active component, then those components may be administered by the same or different routes.

The pharmaceutical formulations of the present invention can be liquids that are suitable for oral, mucosal and/or parenteral administration, for example, drops, syrups, solutions, injectable solutions that are ready for use or are prepared by the dilution of a freeze-dried product but are preferably solid or semisolid as tablets, capsules, granules, powders, pellets, pessaries, suppositories, creams, salves, gels, ointments; or solutions, suspensions, emulsions, or other forms suitable for administration by the transdermal route or by inhalation.

The compounds of the invention can be administered for immediate-, delayed-, modified-, sustained-, pulsed-or controlled-release applications.

In one aspect, oral compositions are slow, delayed or positioned release (e.g., enteric especially colonic release) tablets or capsules. This release profile can be achieved without limitation by use of a coating resistant to conditions within the stomach but releasing the contents in the colon or other portion of the GI tract wherein a lesion or inflammation site has been identified or a delayed release can be achieved by a coating that is simply slow to disintegrate or the two (delayed and positioned release) profiles can be combined in a single formulation by choice of one or more appropriate coatings and other excipients. Such formulations constitute a further feature of the present invention.

Suitable compositions for delayed or positioned release and/or enteric coated oral formulations include tablet formulations film coated with materials that are water resistant, pH sensitive, digested or emulsified by intestinal juices or sloughed off at a slow but regular rate when moistened. Suitable coating materials include, but are not limited to, hydroxypropyl methylcellulose, ethyl cellulose, cellulose acetate phthalate, polyvinyl acetate phthalate, hydroxypropyl methylcellulose phthalate, polymers of metacrylic acid and its esters, and combinations thereof. Plasticizers such as, but not limited to polyethylene glycol, dibutylphthalate, triacetin and castor oil may be used. A pigment may also be used to colour the film. Suppositories are be prepared by using carriers like cocoa butter, suppository bases such as Suppocire C, and Suppocire NA50 (supplied by Gattefosse Deutschland GmbH, D-Weil am Rhein, Germany) and other Suppocire type excipients obtained by interesterification of hydrogenated palm oil and palm kernel oil (C8- C18 triglycerides), esterification of glycerol and specific fatty acids, or polyglycosylated glycerides, and whitepsol (hydrogenated plant oils derivatives with additives). Suspensions are produced by using micronized compounds, and appropriate vehicle containing suspension stabilizing agents, thickeners and emulsifiers like carboxymethylcellulose and salts thereof, polyacrylic acid and salts thereof, carboxyvinyl polymers and salts thereof, alginic acid and salts thereof, propylene glycol alginate, chitosan, hydroxypropylcellulose, hydroxypropylmethylcellulose, hydroxyethylcellulose, ethylcellulose, methylcellulose, polyvinyl alcohol, polyvinyl pyrrolidone, N-vinylacetamide polymer, polyvinyl methacrylate, polyethylene glycol, pluronic, gelatin, methyl vinyl ether-maleic anhydride copolymer, soluble starch, pullulan and a copolymer of methyl acrylate and 2-ethylhexyl acrylate lecithin, lecithin derivatives, propylene glycol fatty acid esters, glycerin fatty acid esters, sorbitan fatty acid esters, polyoxyethylene sorbitan fatty acid esters, polyethylene glycol fatty acid esters, polyoxyethylene hydrated caster oil, polyoxyethylene alkyl ethers, and pluronic and appropriate buffer system in pH range of 6.5 to 8. The use of preservatives, masking agents is suitable. The average diameter of micronized particles can be between 1 and 20 micrometers, or can be less than 1 micrometer. Compounds can also be incorporated in the formulation by using their water-soluble salt forms.

Alternatively, materials may be incorporated into the matrix of the tablet e.g. hydroxypropyl methylcellulose, ethyl cellulose or polymers of acrylic and metacrylic acid esters. These latter materials may also be applied to tablets by compression coating.

Examples of pharmaceutically acceptable disintegrants for oral compositions useful in the present invention include, but are not limited to, starch, pre-gelatinized starch, sodium starch glycolate, sodium carboxymethylcellulose, croscarmellose sodium, microcrystalline cellulose, alginates, resins, surfactants, effervescent compositions, aqueous aluminium silicates and crosslinked polyvinylpyrrolidone..

Examples of pharmaceutically acceptable binders for oral compositions useful herein include, but are not limited to, acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthane resin, alginates, magnesium-aluminum silicate, polyethylene glycol or bentonite.

Examples of pharmaceutically acceptable fillers for oral compositions include, but are not limited to, lactose, anhydrolactose, lactose monohydrate, sucrose, dextrose, mannitol, sorbitol, starch, cellulose (particularly microcrystalline cellulose), dihydro- or anhydro- calcium phosphate, calcium carbonate and calcium sulfate.

Examples of pharmaceutically acceptable lubricants useful in the compositions of the invention include, but are not limited to, magnesium stearate, talc, polyethylene glycol, polymers of ethylene oxide, sodium lauryl sulfate, magnesium lauryl sulfate, sodium oleate, sodium stearyl fumarate, and colloidal silicon dioxide.

Examples of suitable pharmaceutically acceptable odorants for the oral compositions include, but are not limited to, synthetic aromas and natural aromatic oils such as extracts of oils, flowers, fruits (e.g., banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral compositions include, but are not limited to, synthetic and natural dyes such as titanium dioxide, beta-carotene and extracts of grapefruit peel.

Suitable examples of pharmaceutically acceptable sweeteners for the oral compositions include, but are not limited to, aspartame, saccharin, saccharin sodium, sodium cyclamate, xylitol, mannitol, sorbitol, lactose and sucrose. Suitable examples of pharmaceutically acceptable buffers include, but are not limited to, citric acid, sodium citrate, sodium bicarbonate, dibasic sodium phosphate, magnesium oxide, calcium carbonate and magnesium hydroxide.

Suitable examples of pharmaceutically acceptable surfactants include, but are not limited to, sodium lauryl sulfate and polysorbates.

Suitable examples of pharmaceutically acceptable preservatives include, but are not limited to, various antibacterial and antifungal agents such as solvents, for example ethanol, propylene glycol, benzyl alcohol, chlorobutanol, quaternary ammonium salts, and parabens (such as methyl paraben, ethyl paraben, propyl paraben, etc.).

Suitable examples of pharmaceutically acceptable stabilizers and antioxidants include, but are not limited to, ethylenediaminetetriacetic acid (EDTA), thiourea, tocopherol and butyl hydroxyanisole.

Administration may be once a day, twice a day, or more often, and may be decreased during a maintenance phase, e.g. once every second or third day instead of every day or twice a day. The dose and the administration frequency will depend on the clinical signs, which confirm maintenance of the remission phase, with the reduction or absence of at least one or more preferably more than one clinical signs of the acute phase known to the person skilled in the art.

The dosage and treatment duration using the compositions of the invention will depend to some extent on the species and gender of the subject being treated Treatment may need to be carried out for several weeks, e.g. 4 to 20 weeks, before the results desired are achieved.

A dosage of 0.1 to 50 g egg powder, preferably 0.5 to 10 g per day in one or more (especially 2, 3 or 4) doses will generally be preferred. Particularly preferably the dosage will be about 1 to 10 g per day in two to four doses, e.g. morning and evening. The dosage form used in the invention may be any of those mentioned above including emulsions or other semi-liquid dosgaes forms as well as tablets or capsules. In order to administer sufficient active agent without producing an oversize dosage form, it may be necessary to take multiple dosage forms at a time.

Highly preferrred dosages are 1 to 2 g/day. The duration of treatment can vary although an initial period of 3 to 6 weeks is appropriate. After that period, the amount of material could be reduced, e.g. by 50% reduction in the dosage for subsequent weeks unless symptoms persist.

Where the powder is prepared from separated preembryo, these dosages could be reduced by about 60%.

Higher dosages than the 50 g/day mentioned above may be undesirable for prolonged periods, e.g. over two weeks, as the dosage should not be such as to provoke an allergic reaction.

It is advantageous if the medicament of the invention is taken orally.

### Conditions to be treated

The composition of the invention normalises cortisol response in an individual to acute stress. In chronically stressed individuals, this means an elevated up to normal cortisol response compared to those not taking the composition. In non stressed individuals, the opposite effect is observed and the composition would actually allow reduction of cortisol response to acute stress. The composition is especially valuable for chronically stressed individuals.

The maximum increase in cortisol response in chronically stressed individuals can be at least 10%, such as at least 15 % especially at least 25% relative to the cortisol level achieved under otherwise identical conditions without the use of the composition.

The amount of cortisol measured in saliva can range from 5 to 20 nmol/l, e.g. 8 to 14 nmol/l.

Acute stresses which an individual might be subject to include a trauma such as a car accident or other event that could cause post traumatic stress disorder. Acutely stressful events include bereavement, shock, family illness, sitting examinations, interviews, and so on. For those with phobias, dealing with the stress during an exposure to a phobia (e.g. whilst flying) would be acutely stressful.

In this invention, changes in cortisol level were induced using a TSST, a standardized psychosocial stress test. This successfully induced significant changes in cortisol and heart rate. Furthermore, several psychological variables changed in response to the TSST, such as perceived stress and state anxiety as discussed further below.

As expected, high stressed subjects of the placebo group showed a blunted cortisol response in the TSST whereas low stressed subjects in the placebo group showed a normal increase to this challenge. In the treatment group, cortisol levels of subjects with a high impact of chronic stress almost reached levels of low stressed subjects in the test group indicating that they benefit in terms of the composition of the invention raising their cortisol levels up to a normal range in an acute stressful situation. Group differences suggest that the egg powder actively improves adaptation to acute stress by enhancing the endocrine and reducing the subjective stress response.

As an aside, heart rate as indicator of the autonomic nervous system shows less pronounced results but points to a similar direction as the endocrine data.

In sum, these findings suggest that egg composition of the invention restores the ability of chronically stressed subjects to adapt to acute stress.

Whilst the cortisol response is discussed here in connection with exposure to an acute stress, it is envisaged that taking the egg composition of the invention may also serve to improve the general well being of individuals over the long term by normalizing physiological responses to the everyday stresses of life. Thus viewed from another aspect the invention provides a composition comprising a fertilised, incubated avian egg or extract therefrom for use in treating stress in a chronically stressed individual.

### Stress such as Perceived Stress

The invention relates to the reduction in stress, in particular perceived stress in chronically stressed individuals. The invention also relates to the reduction in stress or perceived stress in response to an acutely stressful event.

It must be mentioned at this point that the perception of stress is different from the results presented above in relation to cortisol response to an acute stress. Cortisol release is a measurable endocrine response to a stressor. An individual's perception is a different issue and and rarely correlates with concentration of cortisol.

The composition of the invention has been shown to reduce the perception of stress in an individual especially in response to an acute stress. The observation that the composition of the invention clearly dampened participants' perceived stress was assessed by a visual analogue scale, i.e. the test relies on individuals assessing their own perception objectively on the VAS scale.

By reduction of perceived stress is therefore meant that the perception of stress was reduced, e.g. by at least 10 %.

The maximum increase during the perceived stress test protocol was smaller for the egg powder group compared to the placebo. All subjects benefit of the composition of the invention with respect to their perceived stress, especially in an acute stressful situation.

### Anxiety

Egg powder intake is also associated with a reduction in anxiety, especially less increase of TSST-induced state anxiety, especially in the chronically stressed. The invention also relates to the reduction of anxiety in a response to an acutely stressful event.

The treatment appears to facilitate stressed participants' coping with the test situation and implies the application of the compositions of the invention in general in the treatment of anxiety, especially in chronically stressed individuals.

By reduction in anxiety is meant that the anxiety reported by an individual is preferably at least 10% less than reported under otherwise identical conditions in an individual not taking the composition of the invention. It is especially preferred if the composition of the invention, whether for the reduction in perceived stress, reduction of anxiety or for normalisation of cortisol response is given to deal with an acutely stressful event.

The invention will now be described with reference to the following nonlimiting examples and figures.

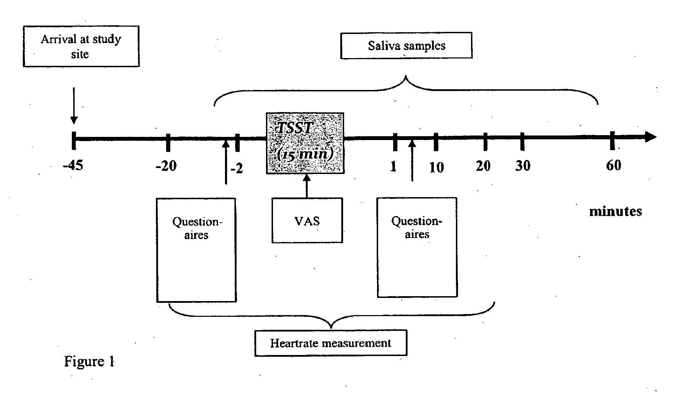

Figure 1 is a timeline for the TSST protocols used to test the composition of the invention.
Figure 2 reports the cortisol response in response to an acute stress for for high and low stress groups.
Figure 3 reports the heart rate in response to an acute stress for for high and low stress groups.
Figure 4 reports anxiety questionnaire response data for individuals taking the composition of the invention relative to a placebo group.
Figure 5 reports stress perception questionnaire date for individuals taking the composition of the invention relative to a placebo group.

### Egg Extract

The fertilised egg extract used was prepared as follows:
The eggs were fertilized and put in an incubator. Incubation took place for 9 - 10 days. The eggs were then checked and unfertilized eggs are removed. The contents of the fertilized and incubated eggs were taken out of the egg shells and the shells are discarded. The liquid parts of the remaining egg were then separated and discarded. The waste may be from the yolk as well as the egg white, as this is the remaining part of the nutrition for the embryo, that has not already been consumed.

The remaining solid fractions represent the preembryo or protoembryo parts of the egg. This isolate is then deep frozen for storage. In order to arrive at the final composition, the blocks of frozen material is milled and freeze dried.

### Placebo

The placebo product contained the following ingredients: rice starch, hydroxypropylmethylcellulose (HPMC), magnesium stearate, colouring agent (yellow iron oxide, black iron oxide, red iron oxide on a lactose carrier) and was produced by Laboratoire GEFA, ZA Bas-Rocomps Route de Noyal-sur-Vilaine, Chateaugiron, France.

Half of the participants in the study were assigned to the first group (active), the other half to the second group (placebo). The investigator was blind to the groups' identity.

40 Participants were recruited on the campus of a local university and via email. Participants were male, between 20 and 50 years old, non-smoking, and healthy. The mean age for both groups was 23.

Questionnaires assessing, *inter alia,* perceived chronic stress and trait anxiety were administered during the first visit. These questionnaires were, inter alia:
1. The Trier Inventory of Chronic Stress (TICS)
2. The Perceived Stress Scale (PSS),
3. The State Trait Anxiety Inventory (trait version: STAI- X2), and
4. The Short Form 12 Health Survey Questionnaire (SF-12).

Study participants then received detailed instructions and a container filled with capsules containing either the test substance or a placebo product. The containers were locked with MEMS TrackCaps, which kept track of the time and date of each opening.

Participants were also given saliva sample collection material for weekly sampling at home and a diary to document their wake-up times on sampling days.

### Treatment Period

During the four weeks leading up to the second visit, participants had to take a daily dose of four capsules: the recommended intake was two capsules with breakfast and two capsules with lunch. Four capsules correspond to a dose of 1680 mg/day egg extract/placebo.

For saliva sample collection cotton swaps were used. Participants had to collect saliva on two consecutive days once a week during the month of substance intake. On each of these days, a first sample had to be taken right after awakening and another sample 30 minutes later, prior to breakfast. These pairs of samples were used to determine the cortisol awakening reaction (CAR) for each week, a reliable marker for chronic stress. Obtained saliva samples were stored frozen or at least cooled.

### Investigation of Treatment Effects: the TSST

After 28 days (four weeks) of substance intake, participants visited the study site again and performed the TSST protocol. They returned the collected saliva samples and handed over the pill bottles with the MEMS TrackCaps, which were used to assess compliance.

The TSST consisted of a resting and anticipation period (45 min.), a test period (15 min.), and a subsequent resting period (60 min.). During the first half of the test period participants had to deliver a free speech. In the second half they had to perform mental arithmetic in front of an audience. Participants also had to fill out a number of questionnaires during their stay, before, during, and after the stress test. The questionnaires were as follows:
1. Perceived Stress Scale
2. SF-12 before the stress test;
3. The State Trait Anxiety Inventory (STAI-X1) assessing state anxiety pre- and post-TSST;
Participants were also asked to rate their degree of perceived stress on a Visual Analogue Scale. This VAS was assessed three times: pre-TSST, in the middle of the TSST, and post-TSST.

Heart rate was recorded from -20 min. to +20 min. in relation to TSST timing by Polar Vantage NV heart rate measurement devices. 10 minutes after the beginning of heart rate measuring subjects were asked to stand up. This serves to avoid confounding orthostatic effects during the TSST measurement. Once the participant returned from the TSST, he remained standing until 10 minutes after the end of the TSST.

The protocol included measures of saliva cortisol (1 pre- and 5 post-measurements at -2 min., +1 min., +10 min., +20 min.,+30 min., and +60 min., respectively). The overall time sequence is illustrated in

After the post-TSST questionnaires participants stayed for another hour during which additional saliva samples are collected.

Free saliva cortisol levels were determined employing an optical density immunoassay (Coated Well EIA, Salimetrics, State College, PA, USA) based on the competition principle. Intra-assay variation of this assay ranges between 3.88 to 7.12%, inter-assay variation between 6.69 to 6.88%.

### Cortisol Response during TSST test

In order to explore the differential effect of the treatment on cortisol levels, saliva cortisol was measured during the TSST test procedure, i.e. an acute stress. For the high stress group, there is a trend for the overall saliva cortisol levels to be higher in the egg powder group than the placebo group. For stressed individuals therefore the egg powder composition encourages an increased cortisol response to an acute stimulus.

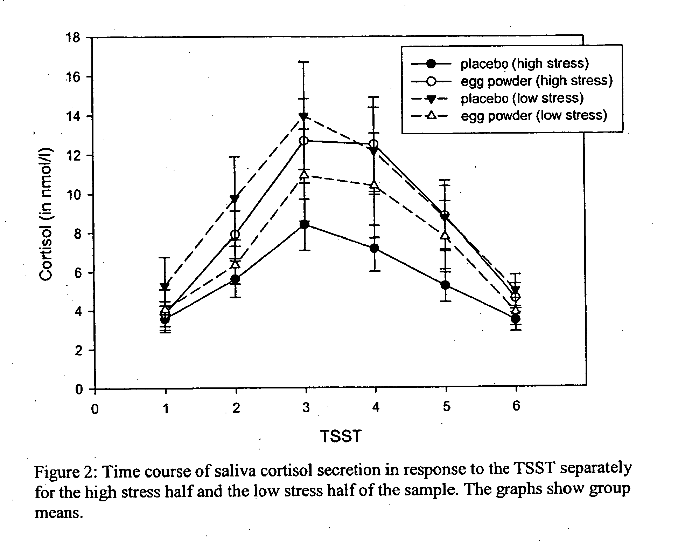

For the low stress group, cortisol levels are lower than those reported in the placebo group, i.e. the low stress group shows a reduction of the cortisol response. This data is represented graphically in figure 2.

### Heart Rate

Heart rate was measured continuously starting 20 minutes prior to the beginning of the TSST, throughout the TSST and 20 minutes after the TSST. Data were aggregated describing different study phases: first, 10 minutes while subjects were sitting, then 10 minutes before the TSST while subjects were standing, 5 minutes of introduction to and preparation for the TSST, 5 minutes of interview during the TSST, 5 minutes of mental arithmetic during the TSST, 10 minutes after the TSST while subjects were standing, and finally 10 more minutes of sitting.

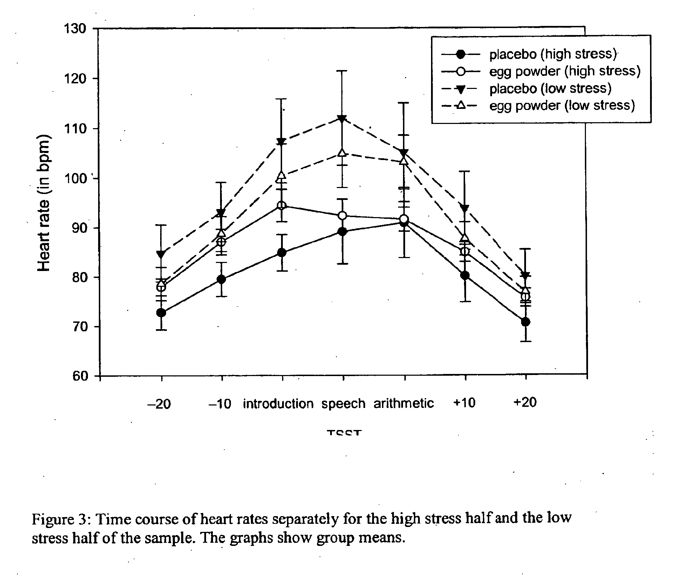

Analyses show that the TSST induced a significant increase of heart rate. As with the cortisol data, additional heart rate analyses were run for the half of the sample with TICS screening scale scores above and below the median (i.e. high and low stress groups), respectively. The data reveals a differential pattern that matches the cortisol data described above. This is shown in figure 3.

### Anxiety

The STAI-X1 anxiety questionnaire was administered on the first visit and twice on the TSST day: once shortly before the TSST, once shortly after the TSST. The TSST intervention yielded a significant higher post-test score. The TSST therefore induced anxiety in individuals.

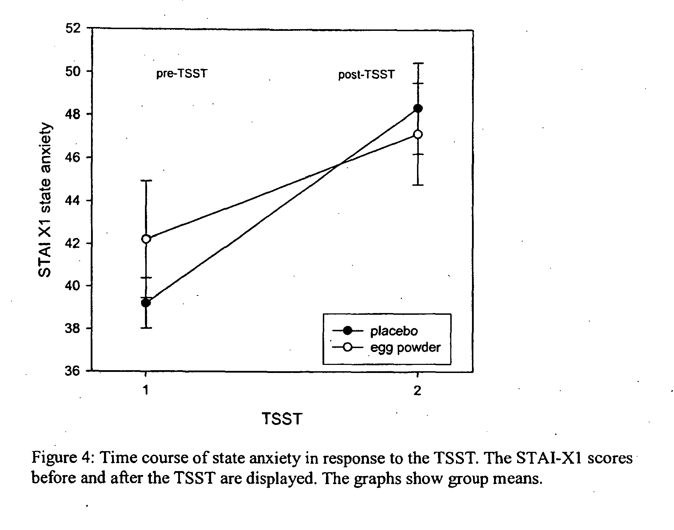

Considering the effects of the composition of the invention, data is shown in Figure 4. The data clearly shows that the increase in anxiety caused by the TSST test is much lower for the test group of the invention than the placebo group. The egg composition can be seen therefore to reduce anxiety relative to a placebo. This result applies to all individuals, in particular in response therefore to an acute stress.

The increase of state anxiety was also tested for group differences. The high stress subsample shows an increase of 2.4 (egg powder) and 12.5 (placebo) scale points, respectively. This suggests that anxiety treatment is especially effective in chronically stressed individuals.

### Perceived Stress

As well as completing appropriate questionnaires, participants rated their perception of the stress test on visual analogue scales three times: before, during, and after the TSST. They rated how high their "stress perception" (VAS 1), during the TSST was.

There were no initial differences in the rating of the TSST between the two experimental groups before the test. The TSST did however induce a significant increase in perceived stress in both groups.

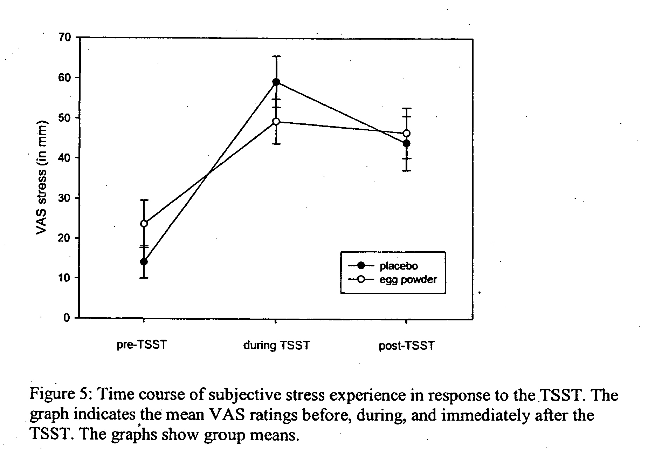

The increase in perceived stress was however reduced during the TSST in individuals who had taken the egg composition. Data are shown in Figure 5.

## Claims

1. A composition comprising a fertilised, incubated avian egg or extract therefrom for use in the treatment of stress such as perceived stress in a chronically stressed individual.

2. A composition comprising a fertilised, incubated avian egg or extract therefrom for use in normalising the cortisol response of a chronically stressed individual to acute stress.

3. A composition comprising a fertilised, incubated avian egg or extract therefrom for use in increasing the cortisol response of an individual to acute stress in a chronically stressed individual.

4. A composition comprising a fertilised, incubated avian egg or extract therefrom for use in the treatment of anxiety in a chronically stressed individual.

5. A composition according to any preceding claim in which the egg or egg from which the extract is formed is in the blastodermal and subsequent preembryonic to protoembryonic stages.

6. A composition according to any preceding claim in which the egg or egg from which the extract is formed is a hen's egg.

7. A composition as claimed in any one of claims 1 to 6 in which the egg or egg from which the extract is formed is a hens egg which has been incubated for 3 to 14 days, especially 9 to 10 days.

8. A composition according to any preceding claim in which the egg extract is a preembryo extract, especially a dried preembryo powder.

9. A composition according to any preceding claim in which the egg powder is present at 5 to 90% by weight, particularly 10 to 80% by weight, especially preferably about 25 to 75% by weight in any composition being administered.

10. A composition as claimed in claim 4 wherein the dosage of fertilised, incubated avian egg or extract therefrom is administered in 1 to 10 g per day units, in two to four doses.

## Patentansprüche

1. Zusammensetzung, umfassend ein befruchtetes bebrütetes Vogelei oder einen Extrakt davon für die Verwendung in der Behandlung von Stress, wie etwa empfundener Stress bei einem chronisch gestressten Individuum.

2. Zusammensetzung, umfassend eine befruchtetes bebrütetes Vogelei oder einen Extrakt davon für die Verwendung in der Normalisierung der Cortisolantwort eines chronisch gestressten Individuums auf akuten Stress.

3. Zusammensetzung, umfassend ein befruchtetes bebrütetes Vogelei oder einen Extrakt davon für die Verwendung zur Steigerung der Cortisolantwort eines Individuums auf akuten Stress bei einem chronisch gestressten Individuum.

4. Zusammensetzung, umfassend ein befruchtetes bebrütetes Vogelei oder einen Extrakt davon für die Verwendung in der Behandlung von Angst bei einem chronisch gestressten Individuum.

5. Zusammensetzung nach einem der vorhergehenden Ansprüche, in der das Ei oder das Ei, aus welchem der Extrakt gebildet wird, in den blastodermalen und den darauf folgenden präembryonalen bis protoembryonalen Stadien ist.

6. Zusammensetzung nach einem der vorhergehenden Ansprüche, in der das Ei oder das Ei, aus dem der Extrakt gebildet ist, ein Hühnerei ist.

7. Zusammensetzung nach einem der Ansprüche 1 bis 6, in der das Ei oder das Ei, aus dem der Extrakt gebildet ist, ein Hühnerei ist, welches für 3 bis 14 Tage, insbesondere 9 bis 10 Tage bebrütet worden ist.

8. Zusammensetzung nach einem der vorhergehenden Ansprüche, in welcher der Eierextrakt ein Präembryo-Extrakt, insbesondere ein getrocknetes Präembryo-Pulver ist.

9. Zusammensetzung nach einem der vorhergehenden Ansprüche, in welcher das Eipulver in 5 bis 90 Gew.-%, vorzugsweise 10 bis 80 Gew.-%, besonders bevorzugt ungefähr 25 bis 75 Gew.-% in jeder Zusammensetzung vorhanden ist, die verabreicht wird.

10. Zusammensetzung nach Anspruch 4, wobei die Dosierung des befruchteten bebrüteten Vogeleis oder dem Extrakt davon in Einheiten von 1 bis 10 g pro Tag in zwei bis vier Dosen verabreicht wird.

## Revendications

1. Composition comprenant un oeuf aviaire fertilisé et incubé ou un extrait de celui-ci pour usage dans le traitement du stress, tel que le stress perçu chez un individu stressé de manière chronique.

2. Composition comprenant un oeuf aviaire fertilisé et incubé ou un extrait de celui-ci pour usage dans la normalisation de la réponse du cortisol d'un individu stressé de manière chronique à un stress aigu.

3. Composition comprenant un oeuf aviaire fertilisé et incubé ou un extrait de celui-ci pour usage dans l'augmentation de la réponse du cortisol d'un individu à un stress aigu chez un individu stressé de manière chronique.

4. Composition comprenant un oeuf aviaire fertilisé et incubé ou un extrait de celui-ci pour usage dans le traitement de l'anxiété chez un individu stressé de manière chronique.

5. Composition selon l'une quelconque des revendications précédentes, dans laquelle l'oeuf ou l'oeuf à partir duquel l'extrait est formé se trouve au stade blastodermique et aux stades pré-embryonnaire à proto-embryonnaire ultérieurs.

6. Composition selon l'une quelconque des revendications précédentes, dans laquelle l'oeuf ou l'oeuf à partir duquel l'extrait est formé est un oeuf de poule.

7. Composition selon l'une quelconque des revendications 1 à 6, dans laquelle l'oeuf ou l'oeuf à partir duquel l'extrait est formé est un oeuf de poule qui a été incubé pendant 3 à 14 jours, en particulier pendant 9 à 10 jours.

8. Composition selon l'une quelconque des revendications précédentes, dans laquelle l'extrait d'oeuf est un extrait pré-embryonnaire, en particulier une poudre pré-embryonnaire séchée.

9. Composition selon l'une quelconque des revendications précédentes, dans laquelle la poudre d'oeuf est présente à raison de 5 à 90 % en poids, en particulier de 10 à 80 % en poids, mieux encore d'environ 25 à 75 % en poids dans toute composition administrée.

10. Composition selon la revendication 4, dans laquelle la dose d'oeuf aviaire fertilisé et incubé ou d'extrait de celui-ci est administrée par unités de 1 à 10 g par jour, en deux à quatre doses.
